# EUROPEAN PATENT APPLICATION

(11) **EP 1 316 312 A1**
(43) Date of publication of application: **04.06.2003**
(21) Application number: 01128265.4
(22) Date of filing: 28.11.2001
(51) Int. Cl.: A61K 35/78, A61P 3/06, A61P 7/00

(54) **Use of the Hibiscus sabdariffa extract in the preparation of a medicament**

(71) Applicant: Universal Biotech Co., Ltd., Ling-Ya Dist., Kaohsiung 802 (TW)
(72) Inventor: Wang, Chau-Jong, South Dist., Taichung 402 (TW)
(74) Representative: TER MEER STEINMEISTER & PARTNER GbR

(57) **Abstract**

Use of the Hibiscus Sabdariffa Extract in the Preparation of a Medicament for Countering Oxidation of Low Density Lipoproteins, Decreasing Triglyceride or Cholesterol Levels in Plasma or Inhibiting Atherosclerosis.

Use of the Hibiscus sabdariffa extract in the preparation of a medicament for countering oxidization of low density lipoproteins, decreasing triglyceride or cholesterol levels in plasma, or inhibiting atherosclerosis.

## Description

### BACKGROUND OF THE INVENTION

### Field of the Invention

The present invention relates to novel applications of the Hibiscus sabdariffa extract in the preparation of a medicament for countering oxidization of low density lipoproteins, reducing cholesterol or triglyceride in plasma or inhibiting atherosclerosis.

### Description of the Related Art

Conventionally, Hibiscus sabdariffa is a local soft drink material and medical herb demonstrating analgesic and antipyretic effects and can be used to cure liver-complaint. The past studies showed that Hibiscus sabdariffa possesses analgesic activity as well as antipyretic and anti-inflammatory action(American Journal of Chinese Medicine, Vol. XXIV, Nos. 3-4, pp. 263-269), antispasmodic potential (Journal of Ethnopharmacology, 31 (1991) 249-257 Elsevier Scientific Publishers Ireland Ltd.), antimutagenic and chemopreventive activity (Food and Chemical Toxicology 37 (1999) 591-601), antioxidant activity(Food and Chemical Toxicology 35 (1997) 1159-1164) and is able to quench the free radicals of 1,1-diphenyl-2-picrylhydrazyl(Food and Chemical Toxicology 38 (2000) 411-416) and lower high blood pressure (Journal of Ethnopharmacology v.65(3) JUNE 1999 P. 231-236).

### SUMMARY OF THE INVENTION

The vessel-related diseases such as apoplexy and heart attack have been the major causes of death in many countries. Though vessel-related diseases are caused by interactions of many factors, atherosclerosis has been the major factor contributing to vessel-related diseases according to the previously published literature.

Viewing of the above, the inventor scanned plants and herbs and finally discovered Hibiscus sabdariffa extract could be applied in decreasing cholesterol or triglyceride levels in plasma, countering oxidization of low density lipoproteins, and inhibiting the abnormal growth of smooth muscle cells or endothelium cells.

Accordingly, it is an object of the present invention to use the Hibiscus sabdariffa extract for the preparation of a medicament for countering oxidization of low density lipoproteins.

Another object of the present invention is the use of the Hibiscus sabdariffa extract for the preparation of a medicament for decreasing cholesterol or triglyceride levels in plasma.

Another object of the present invention is the use of the Hibiscus sabdariffa extract for the preparation of a medicament for inhibiting atherosclerosis.

### BRIEF DESCRIPTION OF THE DRAWINGS

Further objects, features, and advantages of the present invention will become apparent upon consideration of the detailed description of the presentlypreferred embodiments, when taken in conjunction with the accompanying drawings wherein:

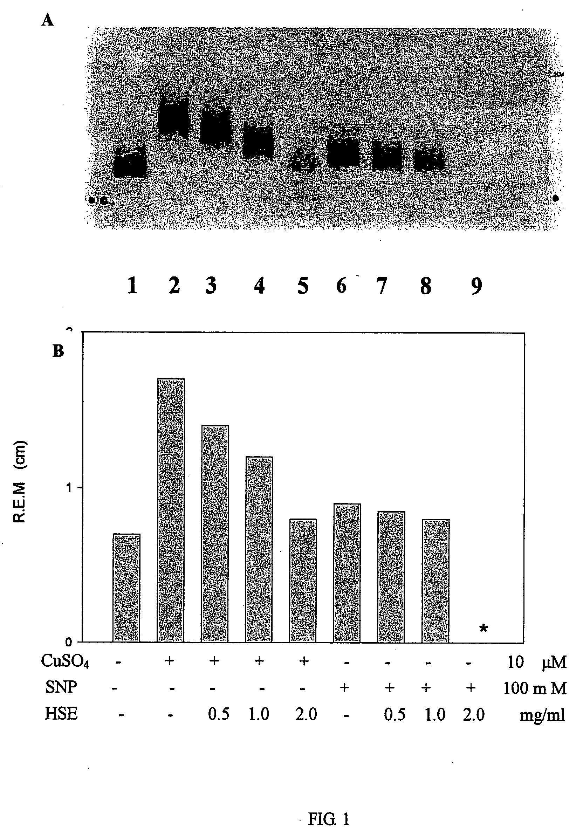

- FIG. 1: is a graph wherein (A) shows Agarose gel electrophoresis of low density lipoprotein (hereinafter referred to as "LDL"). LDL was incubated with CuSO₄ or Sodium Nitroprusside (hereinafter referred to as "SNP") for 24 hours at 37°C. The sample on the gel lanes are follows: lane 1, native LDL ; lane 2, LDL incubated with CuSO₄ ; lanes 3-5, LDL incubated with CuSO₄ in the presence of 0.5 (lane 3), 1 (lane 4) and 2 mg/ml (lane 5) Hibiscus sabdariffa extract (hereinafter referred to as "HSE"); lane 6, LDL incubated with SNP; lanes 7-9, LDL incubated with SNP in the presence of 0.5 (lane 7), 1 (lane 8) and 2 mg/ml (lane 9) HSE; (B) shows Determination of relative electrophoretic mobility. * LDL degradation;
- FIG. 2: is a graph wherein (A) shows Agarose gel electrophoresis of LDL. LDL was incubated with CuSO₄ for 24 hours at 37°C. The sample on the gel lanes are follows: lane 1, native LDL; lane 2, LDL incubated with CuSO₄ ; lanes 3-5, LDL incubated with CuSO₄ in the presence of 0.5 (lane 3), 1 (lane 4), 2 mg/ml (lane 5) HSE; lane 6, LDL pre-incubated with CuSO₄ ; lanes 7-9, LDL pre-incubated with CuSO₄ than addition of 0.5 (lane 7), 1 (lane8) and 2 mg/ml (lane 9) HSE; (B) shows Determination of relative electrophoretic mobility;
- FIG. 3: is a table showing Pre-treative effect of HSE on the CuSO₄ induced lipid peroxidation in LDL. * p< 0.001 ; ** p< 0.00001, compared with CuSO₄ treated LDL group;
- FIG. 4: is a table showing Post-treative effect of HSE on the CuSO₄ induced lipid peroxidation in LDL. * p< 0.01 ; ** p< 0.0001, compared with CuSO₄ treated alone group;
- FIG. 5: is a graph showing Inhibition of Cu²⁺ mediated Apo B fragmentation in LDL by various concentrations of HSE;
- FIG. 6: is a chart comparing the effect of various concentrations of HSE on plasma triglyceride levels in 10 week-period cholesterol fed rabbits. The cholesterol fed diet containing 1.3% cholesterol and 3% lard oil. * p<0.05, compared with the group of cholesterol fed;
- FIG. 7: is a chart comparing the effect of various concentrations of HSE on plasma cholesterol levels in 10 week-period cholesterol fed rabbits. The cholesterol fed diet containing 1.3% cholesterol and 3% lard oil. * p<0.05; ** p<0.01, compared with the group of cholesterol fed.
- FIG. 8: is a chart comparing the effect of various concentrations of HSE on plasma lipid levels in 12 week-period cholesterol fed rats. The cholesterol fed diet containing 1.3% cholesterol and 3% lard oil. * p<0.05; ** p<0.01, compared with the group of cholesterol fed;
- FIG. 9: is a graph showing Internal surface of the thoracic aortas from the five groups of rabbits showing Oil red O stainable lipid deposit;
- FIG. 10: is a graph showing Histological analysis of a representative atherosclerotic lesion from cholesterol-fed rabbits treated for 10 weeks with HSE 0.5% and HSE 1%. Basal diet (A); rabbits fed basal diet with HSE 1% (wt/wt) (B) ; rabbits fed with high cholesterol diet containing 3 % lard oil and 1.3 % cholesterol on basal diet (C and D). The arrow was shown for foam cell (▲) and smooth muscle cell migration (↑) in (C); The calcification core was shown as arrow (▲) in (D); high cholesterol diet fed rabbits and treated with HSE 0.5% (E) and 1% (F) on daily diet;
- FIG. 11: is a table showing Effect of HSE on hepatic function in rabbit and rat. a Abbreviation, ALT, alanine transaminase; AST, asparate aminotransferase; ALP, alkaline phosphatase;
- FIG. 12: is a table showing Effect of HSE on renal function in rabbit and rat. a Abbreviation, BUN, blood urea nitrogen; UA, uric acid.

### DETAILED DESCRIPTION OF THE PREFERRED EMBODIMENT

The preferred embodiment of the present invention relates to the use of the Hibiscus sabdariffa extract in the preparation of a medicament for countering oxidization of low density lipoproteins. The preferred embodiment of the present invention also relates to the use of the Hibiscus sabdariffa extract in the preparation of a medicament for decreasing triglyceride or cholesterol levels in plasma. The preferred embodiment of the present invention further covers the use of the Hibiscus sabdariffa extract in the preparation of a medicament for inhibiting atherosclerosis. According to the present invention it has beneficially found that Hibiscus sabdariffa extract contains natural compounds which have a preventive effect on vessel-related diseases.

The Hibiscus sabdariffa extract is constituted by removing the active constituents from the calyxes or petals of Hibiscus sabdariffa through a water extraction process. The Hibiscus sabdariffa extract may be concentrated or dried if necessary.

The following examples further illustrate the present invention. They are not intended to limit the scope of the invention. Various modifications, alternative constructions and equivalents may be employed without departing from the true spirit and scope of the invention.

### Example 1: Countering Oxidation of LDL

The molecular weight of LDL is smaller when LDL has not been oxidated, therefore, the mobility is greater in the SDS-PAGE experiment under the same potential. Comparatively, the molecular weight of LDL is larger when LDL has been oxidated, therefore, the mobility is lower in the SDS-PAGE experiment under the same potential. After LDL being incubated with 10µM CuSO₄ or 100 mM SNP for 24 hours at 37°C respectively in the presence of various concentrations of HSE , it is observed that the higher the concentration of HSE is, the more significantly the oxidation of LDL is countered. The results are shown in FIG.1.

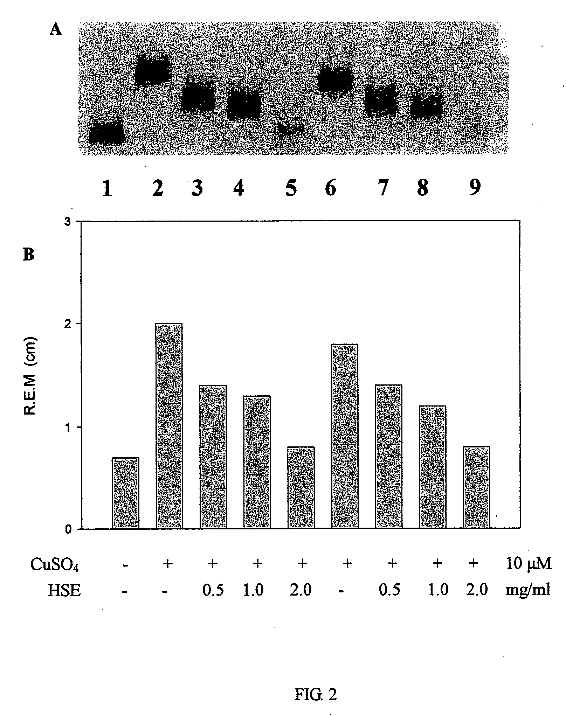

We can learn from the above that the oxidation of LDL incubated with CuSO₄ or SNP will be countered in the presence of HSE. It is also observed that adding HSE after LDL was pre-incubated with CuSO₄ for 24 hours at 37°C is helpful in countering the oxidation of LDL. In addition, the higher the concentration of HSE is, the more significantly the oxidation of LDL is countered. The results are shown in FIG.2.

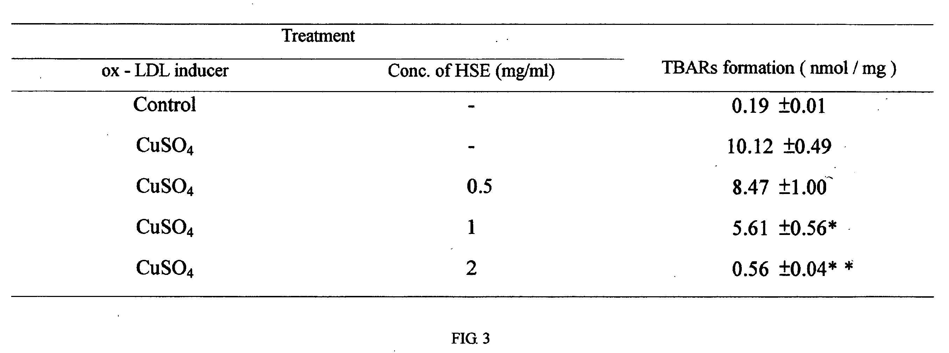

Additionally, LDL were pretreated with various concentrations of HSE for 5 minutes, and then incubated with 10 µM CuSO₄ for 24 hours at 37°C. Comparing with the control group, the measurements of the TBARs indicate that pretreatment with HSE can significantly reduce the formation of TBARs, the group pretreated with 1 mg/dl HSE outperformed the control group by 180% (P<0.01), the group pretreated with 2 mg/dl HSE outperformed the control group by 1800% (P<0.00001). The results are shown in FIG. 3.

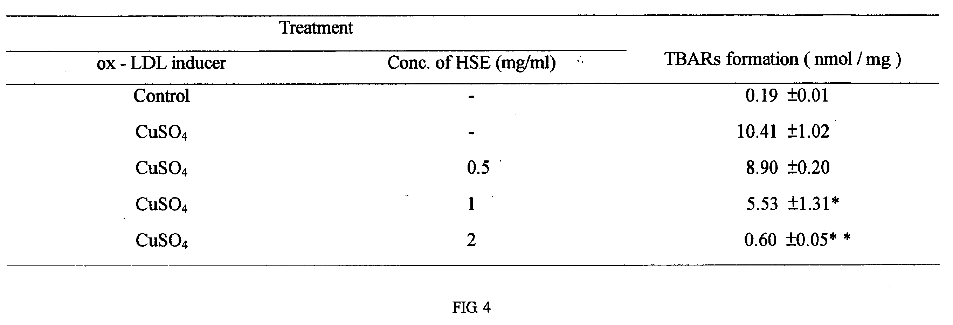

Further, LDL were incubated with CuSO₄ 10 µM for 5 minutes, and then post-treated with various concentrations of HSE for 24 hours at 37°C. The measurements of the TBARs indicate that post-treatment with HSE is able to decrease the formation of TBARs, the group post-treated with 1 mg/dl HSE outperformed the control group by 190% (P<0.01), the group post-treated with 2 mg/dl HSE outperformed the control group by 1700% (P<0.0001). The results are shown in FIG. 4.

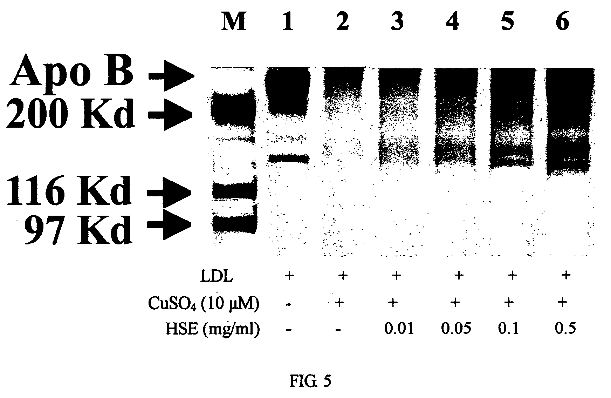

Cu²⁺ may induce the oxidation of LDL and then cause Apo B fragmentation in LDL. LDL (120 mg/ml) was incubated with 10 mM CuSO₄ at 37°C in the presence of HSE for 4 hours. After the incubation, EDTA (final concentration 1 mM) was added to prevent any further oxidation. Approximately 6 mg protein of the LDL was applied to SDS-PAGE (3-15% gradient). After the electrophoresis, each spot was stained with Coomassie Brilliant blue R250. M, standard molecular weight markers. It is observed that the higher the concentration of HSE is, the less the Apo B fragmentation in LDL is caused, i.e., the higher the concentration of HSE is, the more significantly the oxidation of LDL is countered. The results are shown in FIG. 5.

### Example 2: Decreasing Triglyceride or Cholesterol Levels in Plasma

30 New Zealand rabbits (2000-2200g) and 24 Sprague-Dawley rats (200-220g) were used for the following experiments. The rabbits were split into five groups, each rabbit was cooped in a cage and fed with 150g diet every day for 10 weeks. The diet of 3 groups contained 1.3% cholesterol and 3% lard oil (i.e. cholesterol-fed diet) in order to induce atherosclerosis. The diet of the other 2 groups contained 1% and 0.5% HSE respectively. The rats were split into four groups, 6 rats were cooped in a cage and each rat was fed with 25g diet every day for 12 weeks. The diet of 2 groups contained 1.3% cholesterol and 3% lard oil in order to induce atherosclerosis. The diet of the other 2 groups contained 1% and 2% HSE respectively.

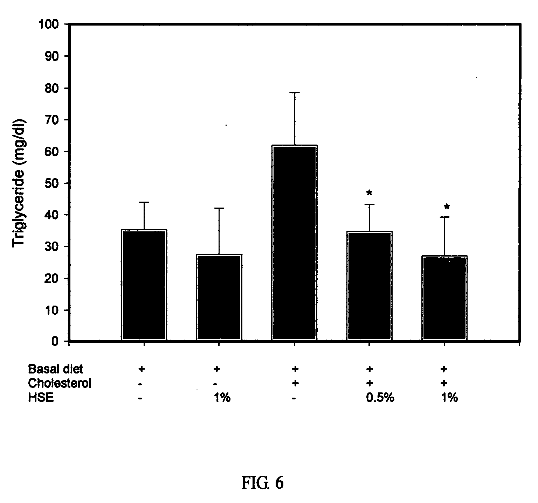

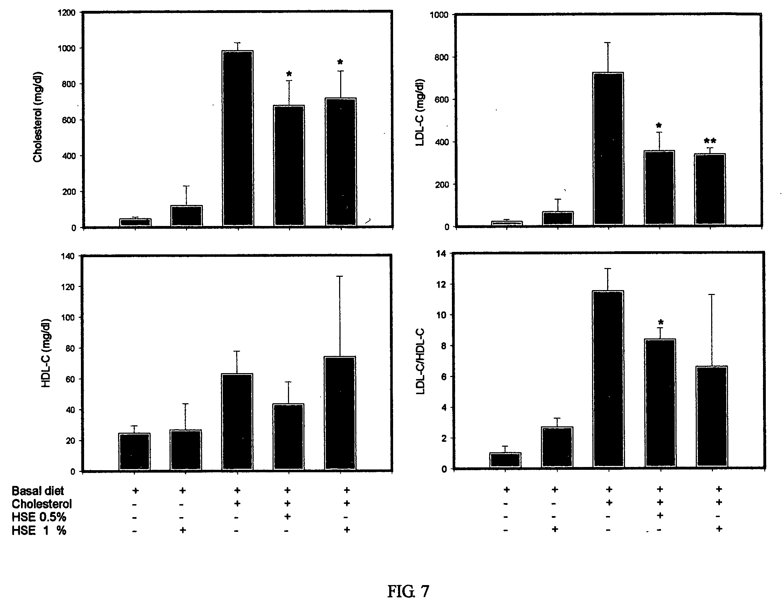

After having been fed for ten weeks, the rabbits were anesthetized using sodium pentothal (16mg/kg, i.v.) and then sacrificed. The blood was collected in the EDTA-coated tubes, and then centrifugated for the purpose of analyzing plasma lipid levels including plasma triglyceride levels and plasma cholesterol levels. The study shows adding HSE in the cholesterol-fed diet is capable of decreasing plasma triglyceride levels (p<0.05). The results are shown in FIG. 6. Cholesterol-fed diet fed rabbits raised their plasma cholesterol levels up to 8.4 folds, their LDL-C levels up to 10.5 folds, LDL-C/HDL-C ratios up to 4.4 folds from the original levels; adding 0.5% HSE in the cholesterol-fed diet is capable of decreasing plasma cholesterol levels, LDL-C levels and LDL-C/HDL-C ratios (p<0.05), adding 1% HSE in the cholesterol-fed diet is capable of decreasing plasma cholesterol levels, LDL-C levels and LDL-C/HDL-C ratios (p<0.01). The results are shown in FIG.7.

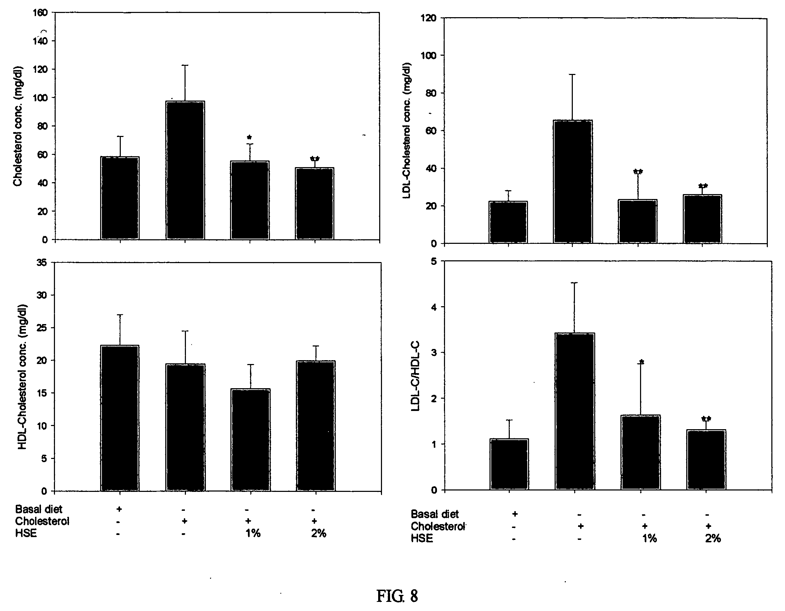

The rats were sacrificed after having been fed for twelve weeks. The blood was collected in the EDTA-coated tubes, and then centrifugated for the purpose of analyzing plasma cholesterol levels. It is observed that Cholesterol-fed diet fed rats raised their plasma cholesterol levels up to 1.6 folds, their LDL-C levels up to 3 folds, LDL-C/HDL-C ratios up to 2.6 folds from the original levels; adding 1% HSE in the cholesterol-fed diet is capable of decreasing plasma cholesterol levels, LDL-C levels and LDL-C/HDL-C ratios (p<0.05), adding 2% HSE in the cholesterol-fed diet is capable of decreasing plasma cholesterol levels, LDL-C levels and LDL-C/HDL-C ratios (p<0.01). The results are shown in FIG.8. Thus, it is further proven that the HSE can decrease triglyceride or cholesterol levels in plasma.

### Example 3: Inhibition of Atherosclerosis

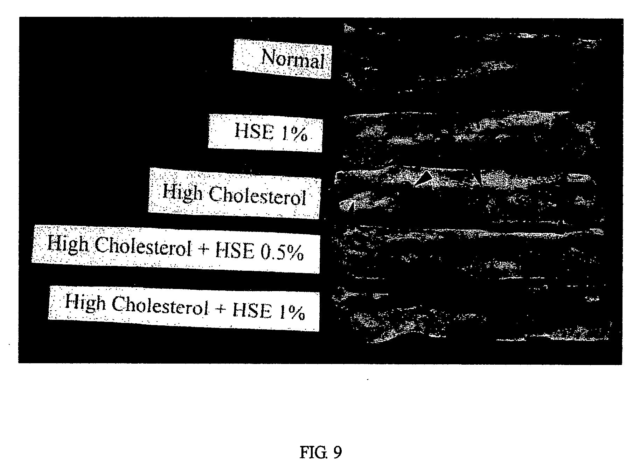

Lipid deposit in the endothelium cells of arteriae often leads to atherosclerosis, that is, if the lipid deposit in the endothelium cells of arteriae has reached certain amount, then it is quite possible that atherosclerosis may occur. In this study, we stained the chest arteriae of the rabbits recited in example 2 to discover the conditions of lipid deposit. This study shows there is much deposit in the arteriae' endothelium cells of those rabbits fed with Cholesterol-fed diet, however, the lipid deposit substantially decreased accompanying the addition of HSE. The results are shown in FIG.9.

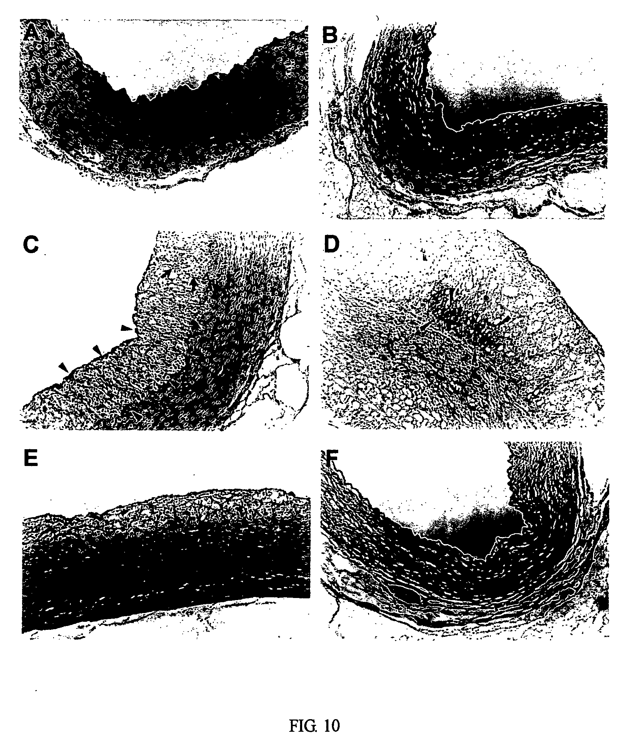

Furthermore, we pathologically biopsied the arteriae of the rabbits recited in example 2 and found that no foam cells formed in the arteriae of the rabbits fed with Cholesterol-fed diet added with 0.5% HSE or 1% HSE and fed with Basil diet. In addition, no migration of smooth muscle cells or formation of calcification core was found. The results are shown in FIG. 10.

### Example 4: No Side-Effect on Hepatic Function and Renal Function

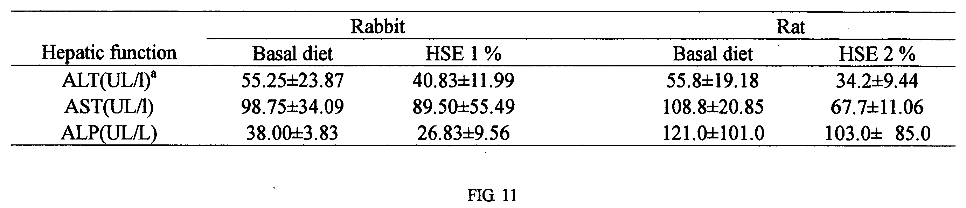

The rabbits were fed with Basil diet and Basil diet added with 1% HSE respectively, The rats were fed with Basil diet and Basil diet added with 2% HSE respectively. It is observed that there is no substantial difference between the measurements of ALT, AST, ALP of both groups. Thus, it is proven that HSE does not make harm to hepatic function. The results are shown in FIG. 11.

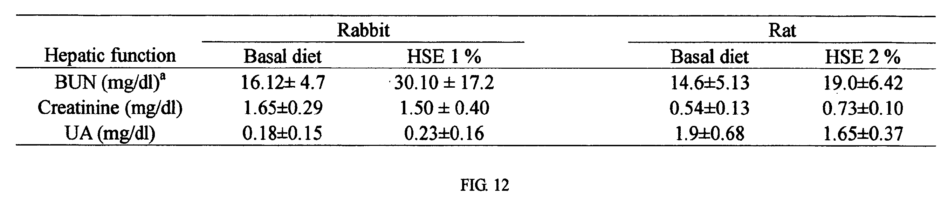

The rabbits were fed with Basal diet and Basal diet added with 1% HSE respectively, The rats were fed with Basal diet and Basal diet added with 2% HSE respectively. It is observed that there is no substantial difference between the measurements of BUN, AST, ALP of both groups. Thus, it is proven that HSE does not make harm to renal function. The results are shown in FIG. 12.

## Claims

1. Use of the Hibiscus sabdariffa extract in the preparation of a medicament for countering oxidization of low density lipoproteins.

2. Use of the Hibiscus sabdariffa extract in the preparation of a medicament for decreasing triglyceride levels in plasma.

3. Use of the Hibiscus sabdariffa extract in the preparation of a medicament for decreasing cholesterol levels in plasma.

4. Use of the Hibiscus sabdariffa extract in the preparation of a medicament for inhibiting atherosclerosis.
